# EUROPEAN PATENT SPECIFICATION

(11) **EP 4 298 019 B1**
(45) Date of publication and mention of the grant of the patent: **26.03.2025**
(21) Application number: 21707965.6
(22) Date of filing: 23.02.2021
(51) Int. Cl.: B64F 1/36, A61L 9/20, A61L 2/14, A61L 2/20, A61L 9/22, A61L 2/08

(54) **APPARATUS FOR CONDITIONING AIR IN AN AIRCRAFT CABIN ON THE GROUND AND FOR SANITIZING SURFACES OF THE CABIN**
VORRICHTUNG ZUR KONDITIONIERUNG VON LUFT IN EINER FLUGZEUGKABINE AUF DEM BODEN UND ZUR DESINFEKTION VON OBERFLÄCHEN DER KABINE
APPAREIL POUR LE CONDITIONNEMENT DE L'AIR DANS UNE CABINE D'AÉRONEF AU SOL ET POUR LA DÉSINFECTION DES SURFACES DE LA CABINE

(43) Date of publication of application: 03.01.2024
(73) Proprietor: Lebrun - Nimy, 7020 Nimy (BE)
(72) Inventor: MAHIEU, Maxime, 7020 Nimy (BE); URBAIN, Claudy, 7020 Nimy (BE); DECOUX, Laurent, 7020 Nimy (BE)
(74) Representative: Connor, Marco Tom
(86) International application number: PCT/EP2021/054450
(87) International publication number: WO 2022/179679

(56) References cited:
- DE-A1- 102005 003 923
- KR-B1- 101 277 502
- US-A1- 2009 311 138
- US-A1- 2010 003 164

## Description

### TECHNICAL FIELD

The present invention concerns a pre-conditioned air (PCA) device for conditioning the air in an interior of a cabin of an aircraft parked on the ground and, at the same time, for sanitizing and possibly disinfecting the air and surfaces in the interior of the cabin. The PCA-device of the present invention is particularly advantageous in that it offers an additional function of sanitizing the air and surfaces inside the cabins of aircraft without changing the routine in application for decades of pre-conditioning the air inside the cabins of aircraft. The pre-conditioned air is treated before being injected into the cabin such as to carry reactive oxygen species (ROS) including dry hydrogen peroxide (DHP) at concentrations compatible with the presence of humans in the cabin during the injection of the thus treated pre-conditioned air. State-of-the-art equipment for injecting the pre-conditioned air can be used in the present invention with a simple and inexpensive modification of the equipment.

### BACKGROUND OF THE INVENTION

When aircraft are parked on the ground, the air inside the cabin needs be conditioned, heated in winter and cooled in summer. When the engines of an aircraft on the ground are off, the energy required for these operations can be provided by the auxiliary power unit (APU) present in most large aircraft. The APU is an auxiliary turbine which is noisy and very expensive to run as it consumes substantial amounts of kerosene with related CO₂ emissions.

To reduce CO₂ emissions, most international airports are equipped with pre-conditioned air (PCA) devices for conditioning the air in an interior of a cabin of an aircraft parked on the ground with their APU's switched off. Pre-conditioned air systems provide an external supply of conditioned air to cool, ventilate and heat the cabins of parked aircraft. An example of pre-conditioned air system is described in KR101277502. To allow complete switching off the APU, electric power must also be supplied externally. For this reason, many PCA-devices are combined with an AC-electrical power supply typically at 400 Hz. To provide a parked aircraft with conditioned air and with electric power, the APU would be running on average one hour for a narrow-body aircraft and over 1.5 hours for a wide-body aircraft. Having both power and air at the parking location would cut 90% of the APU usage resulting in both less pollution and substantial cost savings.

With the tight schedules on the ground, it becomes difficult for the cleaning teams to properly clean and disinfect all surfaces inside the cabin between the disembarking of the inbound passengers and the embarking of the outbound passengers. With the present crisis of COVID 19, the regulations for sanitizing and disinfecting the interiors of cabins have been tightened drastically. Yet no additional time is granted to the cleaning teams for meeting these new regulations.

In continuation, the term "*sanitizing*" (and derivatives) refers to lowering the number of germs on a surface to a safe level, according to pre-defined standards. The term "*disinfecting*" (and derivatives) refers to killing nearly 100 percent of germs on surfaces or objects. Disinfecting is generally slightly stronger than sanitizing. For example, the U.S. Environmental Protection Agency (EPA) sets the levels of germs killing to 99.9% for sanitizing and to 99.999% for disinfecting. While this difference might seem minimal, it can make a substantial difference in reducing the spread of an infection. The term "*sterilizing*" (and derivatives) refers to destroying or eliminating all forms of microbial life (i.e., 100% of germs). The term "*cleaning*" (and derivatives) refers to removing dirt and other impurities from a surface. Each of sanitizing, disinfecting, and sterilizing does kill germs but does not necessarily clean dirty surfaces.

Systems have been proposed for sanitizing a seat surface in an aircraft cabin by irradiation of the seat surface with UV-light. For example, EP2772272 describes an autonomous trolley configured for moving along an aisle of an aircraft, with unfolded articulated arms extending over the seats on either side of the aisle. Each arm is equipped with sources of UV-C light oriented towards the seats. Only the seats are treated, not e.g., the overhead stowage bins., and only the seats surfaces exposed to the UV-C light are effectively sanitized. If a magazine lies on a seat, sanitization will be incomplete. The level of sanitization strongly depends on the distance of the surface to be treated from the source of UV-C light. Furthermore, exposure to UV-C light is harmful to human eyesight, Finally, when running, the trolley is on the way of the cleaning teams, disrupting their cleaning routines. US2009311138 describes a method of sanitizing a passenger cabin comprising injecting ozone in the cabin to create an ozone enriched atmosphere in the cabin. The ozone concentration is such that the cabin must be evacuated and sealed.

The use of hydrogen peroxide (H₂O₂) for treating different environments has been described in the art but to our knowledge, never for sanitizing a cabin of a parked aircraft. For example, WO2016176486 describes the use of dry hydrogen peroxide (DHP) for preserving fruits and vegetables enclosed in an enclosed environment such as a restaurant, barn, or the like. US20180192619 describes the use of DHP for sanitizing poultry eggs, chicks, and birds in hatcheries and poultry farms. US7354551 describes a system for decontaminating a room such as a hotel room including a vapour generator which supplies a decontaminant vapour, such as hydrogen peroxide vapour to the room. All these systems, however, require injecting continuously hydrogen peroxide to maintain a constant concentration thereof in the air. This is, however, not possible with an aircraft cabin since the aircraft remains on the ground for a very limited time only during a landing-taking off cycle (on average one hour for a narrow-body aircraft and over 1.5 hours for a wide-body aircraft).

The present invention proposes a solution to the problem of reaching the ever-higher sanitizing and disinfecting standards imposed to airlines, without increasing the short time an aircraft remains parked on the ground within a cycle of disembarking and embarking of passengers, and without disrupting neither the ground operations nor the routine of the cleaning teams during the even shorter time the aircraft is empty of passengers. With the present invention, the passengers and cleaning teams are not even aware that the air and surfaces of the aircraft's cabin are being sanitized as they are cleaning the cabin. These and other advantages of the present invention are presented in continuation.

### SUMMARY OF THE INVENTION

The present invention is defined in the appended independent claims. Preferred embodiments are defined in the dependent claims. In particular, the present invention concerns a pre-conditioned air (PCA-) device for conditioning air in an interior of a cabin of a parked aircraft and for ventilating and sanitizing an air and surfaces in the interior of the cabin, the PCA-device comprising,
- an air pre-conditioning (PCA-) unit comprising,
   ∘ a PCA-inlet configured for admitting ambient air from a surrounding environment, the ambient air being at an ambient temperature (Ta), at an ambient relative humidity (RHa), and an ambient pressure (P),
   ∘ an air filtration section located downstream of the PCA-inlet,
   ∘ an air conditioning section located downstream of the PCA-inlet,
   ∘ a PCA-outlet located downstream of both air filtration and air conditioning sections, and configured for dispensing a pre-conditioned air stream of pre-conditioned air at a pre-conditioned temperature (Tc ≠ Ta) different from the ambient temperature (Ta), at a pre-conditioned relative humidity (RHc > 0) having a positive value, and at a pre-conditioned pressure (Pc > Pa) greater than the ambient pressure (Pa),
- an air distribution ducting comprising a flexible portion, the air distribution ducting extending from a proximal end in fluid communication with the PCA-outlet, to a distal end provided with a coupling head (4) configured for being fluidly coupled to an internal air circulation duct of an aircraft,
- wherein the PCA-device defines a continuous air pathway extending from the PCA-inlet to the coupling head,

The PCA-device comprises a photo catalytic oxidation unit located downstream of the PCA-inlet, preferably at or downstream of the PCA-outlet and intersecting the continuous air pathway, the photo catalytic oxidation unit being configured for producing a peroxided air) by formation of dry hydrogen peroxide at a concentration below 1 ppm in the pre-conditioned air dispensed out of the PCA-outlet, wherein the photo catalytic oxidation unit comprises,
- a source of ultraviolet (UV-) light configured for irradiating a UV-volume with UV-light, and
- a catalyst located within the UV-volume and intersecting the continuous air pathway.

The photo catalytic oxidation unit preferably intersects the pre-conditioned air stream of pre-conditioned air) and is preferably located at or directly adjacent to the PCA-outlet.

In a preferred embodiment, the catalyst is coated on a surface of an air-permeable structure intersecting the continuous air pathway. The air-permeable structure can be formed by a mesh or a grid, both comprising openings, or a series of hollow tubes assembled in a staggered, honeycomb structure defining openings. At least a selection of the openings of the mesh, grid, or hollow tubes is oriented substantially parallel to the continuous air pathway.

The catalyst can be a metal oxide, preferably titanium dioxide or tungsten dioxide, or a mixture of metal oxides including titanium dioxide. The UV-light can have a wavelength comprised between 200 and 400 nm, preferably the UV-light is UV-A light of wavelength comprised between 320 and 400 nm. The dry hydrogen peroxide concentration in the peroxided air (5p) thus produced can be not more than 0.06 ppm, preferably not more than 0.04 ppm, and is at least 0.01 ppm, preferably at least 0.02 ppm. the peroxided air (5p) further comprises reactive oxygen species (ROS) including one or more of ˙O₂⁻ and ˙OH.

The PCA-unit can either be,
- a fixed system preferably coupled to a passenger-boarding bridge (12), or
- a mobile system which can be brought to an aircraft parked at a position remote from a terminal.

The PCA-unit can be a combined system configured for also supplying alternative current at a frequency of preferably 400 Hz.

The present invention also concerns a method for sanitizing an air and surfaces of an interior of a cabin of a parked aircraft (12) and for conditioning the air in the interior of the cabin, the method comprising,
- providing a PCA-device according to any one of the preceding claims,
- fluidly coupling the coupling head (4) to an internal air circulation duct of the aircraft
- drawing ambient air (5a) into the PCA-device unit (1) through the PCA-inlet (1i),
- conditioning the ambient air inside the PCA-device unit (1) and dispensing a pre-conditioned air stream of pre-conditioned air (5c) out of the PCA-outlet at the pre-conditioned temperature (Tc), at the pre-conditioned relative humidity (RHc), and at the pre-conditioned pressure (Pc),
- leading the pre-conditioned air stream along the air distribution ducting (3) into the internal air circulation duct of the aircraft,
**characterized in that,** the method comprises the following steps,
- activating the source of UV-light (2uv) and ensuring that air flowing along the continuous air pathway contacts the catalyst (2c) irradiated by the UV-light within the UV-volume, to form the pre-conditioned air stream of pre-conditioned air (5c) comprising not more than 1 ppm of dry hydrogen peroxide, and
- allowing the peroxided air (5p) to circulate into the cabin for a given time to sanitize and disinfect the air and surfaces it contacts.

### BRIEF DESCRIPTION OF THE FIGURES

For a fuller understanding of the nature of the present invention, reference is made to the following detailed description taken in conjunction with the accompanying drawings in which:
**Figure 1(a)** shows a parked aircraft coupled to a fixed PCA device through an air distribution ducting partly fixed to a passenger boarding bridge.
**Figure 1(b)** shows a parked aircraft coupled to a fixed PCA device.
**Figure 1(c)** shows a parked aircraft coupled to a mobile PCA device.
**Figures 2(a) & 2(b)** illustrate schematically the photo-catalytic formation of reactive oxygen species (ROS) with two different set-ups.
**Figures 3(a) to 3(c)** show three embodiments of photo catalytic oxidation units according to the present invention.
**Figure 4(a)** shows an example of photo catalytic oxidation unit of the type illustrated in Figure 3(a),
**Figures 4(b) to 4(e)** show different embodiments of air-permeable structures suitable for supporting a coating of catalyst.
**Figures 4(f) & 4(g)** show two examples of air-permeable structures coated with a catalyst.
**Figure 5(a)** shows an example of PCA-device of the prior art.
**Figures 5(b) to 5(d)** show different embodiments of PCA-devices according to the present invention.

### DETAILED DESCRIPTION OF THE INVENTION

The present invention concerns a pre-conditioned air (PCA-) device for conditioning air in an interior of a cabin of a parked aircraft (12) and for sanitizing an air and surfaces in the interior of the cabin. The PCA-device comprises an air pre-conditioning (PCA-) unit (1) and an air distribution ducting (3).

The air pre-conditioning (PCA-) unit (1) comprises,
- a PCA-inlet (1i) configured for admitting ambient air (5a) from a surrounding environment, the ambient air being at an ambient temperature (Ta), at an ambient relative humidity (RHa), and an ambient pressure (P), all depending on the instant meteorological conditions,
- an air filtration section (1f) located downstream of the PCA-inlet,
- an air conditioning section (1ac) located downstream of the PCA-inlet,
- a PCA-outlet (1o) located downstream of both air filtration and air conditioning sections, and configured for dispensing a pre-conditioned air stream of pre-conditioned air (5c) at a pre-conditioned temperature (Tc ≠ Ta) different from the ambient temperature (Ta), at a pre-conditioned relative humidity (RHc > 0) having a positive value, and at a pre-conditioned pressure (Pc > Pa) greater than the ambient pressure (Pa),

The air distribution ducting (3) comprises a flexible portion (3f) and may or may not comprise rigid portions (3r), depending on whether the PCA-unit is mobile or fixed. The air distribution ducting extends from a proximal end in fluid communication with the PCA-outlet (1o), to a distal end provided with a coupling head (4) configured for being fluidly coupled to an internal air circulation duct of an aircraft. The PCA-device defines a continuous air pathway extending from the PCA-inlet (1i) to the coupling head (4).

The present invention differs from the prior art in that the PCA-device comprises a photo catalytic oxidation unit (2) located anywhere downstream of the PCA-inlet (1i), preferably at or downstream of the PCA-outlet (1o). The photo catalytic oxidation unit (2) is positioned such as to intersect the continuous air pathway. The photo catalytic oxidation unit is configured for producing a peroxided air (5p) by formation of dry hydrogen peroxide at a concentration below 1 ppm in the pre-conditioned air (5c) dispensed out of the PCA-outlet.

Dry hydrogen peroxide (DHP) of formula H₂O₂ is a gas. It is not a vapour from aqueous hydrogen peroxide solutions. DHP behaves like oxygen and nitrogen, diffusing through the air. At concentrations below 1 ppm, DHP is effective for extremely high microbial reduction while being very safe to humans.

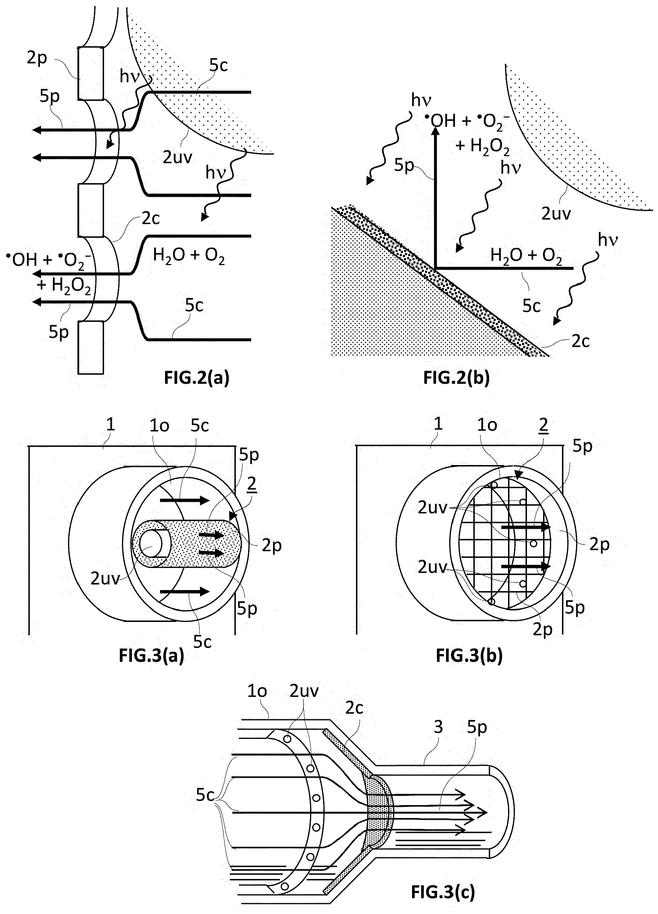

As illustrated in Figures 2(a)&2(b), 3(a) to 3(c), and 4(a), the photo catalytic oxidation unit (2) comprises,
- a source of ultraviolet (UV-) light (2uv) configured for irradiating a UV-volume with UV-light, and
- a catalyst (2c) located within the UV-volume and intersecting the continuous air pathway.

The catalyst is preferably coated on a substrate. The substrate can be air-permeable or not.

### PHOTOCATALYTIC PRODUCTION OF REACTIVE OXYGEN SPECIES (ROS)

Reactive oxygen species (ROS) are chemical molecules with one unpaired electron, formed due to the electron acceptability of O₂. ROS are therefore highly reactive. Examples of ROS include peroxides, in particular hydrogen peroxide, superoxide, hydroxyl radical, and the like.

The reduction of molecular oxygen (O₂) produces superoxide (˙O₂⁻), which is the precursor of most other reactive oxygen species (O₂ + e⁻ → ˙O₂⁻). Dismutation of superoxide produces hydrogen peroxide (H₂O₂) (2 H⁺ + ˙O₂⁻ + ˙O₂⁻ → H₂O₂ + O₂). Hydrogen peroxide in turn may be partially reduced, thus forming hydroxide ion and hydroxyl radical (˙OH), or fully reduced to water (H₂O₂ + e⁻ → HO⁻ + ˙OH and 2 H⁺ + 2 e⁻ + H₂O₂ → 2 H₂O).

ROS can be produced by photocatalysis, which is a photoreaction accelerated by the presence of a catalyst (2c). In presence of oxygen and water present in the air, the catalyst (2c) creates electron-hole pairs, which generate free radicals (e.g. hydroxyl radicals: ˙OH and superoxides ˙O₂⁻) able to undergo secondary reactions.

The catalysts are generally transition metal oxides and semiconductors, as they possess a band gap where no energy level is available to promote recombination of an electron and hole produced by photoactivation in the solid. When a photon with energy equal to or greater than the materials band gap is absorbed by the semiconductor, an electron is excited from the valence band to the conduction band, generating a positive hole in the valence band: MO + hv → MO (h⁺ + e⁻), wherein MO is a metal oxide, h⁺ and e⁻ are a hole and an electron, and hv is optical energy. Such a photogenerated electron-hole pair is termed an exciton. Upon exposure to oxidants, the excited electrons react to produce reduced products, and upon exposure to reductants, the generated holes react to produce oxidized products at the surface of catalyst (2c) according to the following schemes.

| *Photocatalytic oxidative reactions* | *Photocatalytic reductive reactions* |
|---|---|
| h⁺ + H₂O → H⁺ + ˙OH | e⁻ + O₂ → ˙O₂⁻ |
| 2 h⁺ + 2 H₂O → 2 H⁺ + H₂O₂ | ˙O₂⁻ + H₂O + H⁺ → H₂O₂ + O₂ |
| H₂O₂→ 2 ˙OH | H₂O₂ → 2 ˙OH |

### PHOTO-CATALYTIC UNIT (2)

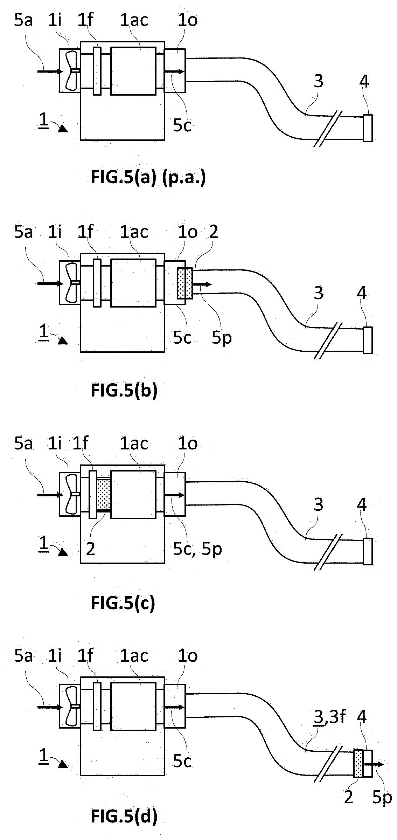

As illustrated in Figures 2(a)&2(b), 3(a) to 3(c), and 4(a) the photo-catalytic unit (2) comprises a source of UV-light (2uv) and a catalyst (2c) positioned such as to be irradiated by UV-light and as to intersect the continuous air pathway followed by the air. Figures 5(b) to 5(d) show different embodiments for positioning the photo-catalytic unit (2) within the PAC-device. In a preferred embodiment shown in Figure 5(b), the photo-catalytic unit (2) intersects the pre-conditioned air stream of pre-conditioned air (5c) and is preferably located partly or entirely at the PCA-outlet (1o). It can also be positioned directly adjacent to the PCA-outlet (1o), either upstream or downstream thereof.

As shown in Figure 5(c) the photo-catalytic unit (2) can be located within the PCA-unit (1), between the PCA-inlet (1i) and the PCA-outlet (1o). Finally, the photo-catalytic unit (2) can be positioned in the air distribution ducting (3), preferably at or near the coupling head (4). Since the photo-catalytic unit (2) requires power to activate the source of UV-light, this embodiment is best suited with combo-types of PCA-devices combining PCA- and power-supplies to the aircraft, such that the photo-catalytic unit (2) can be powered at or close to the distal end of the air distribution ducting (3). This solution has the drawbacks of a trickier power supply and heavier coupling head, more difficult to handle by an operator. It has the advantage that the ROS are produced directly at an inlet of the internal air circulation duct of the aircraft, such that short lived ROS's can reach the interior of the cabin.

The source of UV-light preferably emits UV-light having a wavelength comprised between 200 and 400 nm. Preferably the UV-light is UV-A light of wavelength comprised between 320 and 400 nm. UV-B or UV-C lights can also be used without danger, since the photo-catalytic unit (2) is enclosed within the PCA-device and people are never exposed to the UV-light. The source of UV-light (2uv) can be a UV-LED (light emitting diode), or a UV-laser, or a UV-fluorescent lamp tube.

The catalyst (2c) is preferably coated on a support. Figures 2(b) and 3(c) illustrate examples of a catalyst (2c) coated on a surface of a wall. Figure 3(c) illustrates an embodiment wherein the catalyst (2c) is coated on a restriction formed between the PCA-outlet (1o) and the air distribution ducting (3). UV-lamps (2uv) are oriented towards the coated tapering walls against which the pre-conditioned air (5c) flow is deviated. Baffles coated with a catalyst (2c) can also be positioned across the continuous air pathway (not shown). Care must be taken to not create excessive pressure drops with too many obstacles intersecting the continuous air pathway.

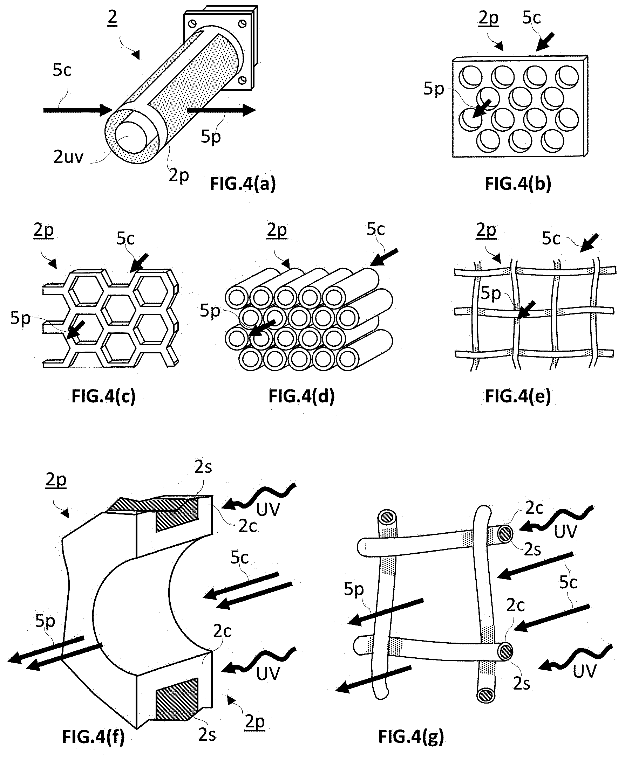

In a preferred embodiment illustrated in Figures 2(a) to 4(a) and 3(b), the surface on which the catalyst is coated belongs to an air permeable structure (2p) intersecting the continuous air pathway. As shown in Figures 3(b), 4(b)&4(c), the air-permeable structure (2p) can be formed by a grid comprising openings of any geometry. The air-permeable structure (2p) can be a mesh, as shown in Figures 3(b) and 4(e). Alternatively, it can be formed by a series of hollow tubes assembled or stacked in a staggered, honeycomb-like structure defining openings as shown in Figure 4(d). The openings of the air-permeable structure (2p) should be oriented such as to optimize a contact of the air flow with the catalyst (2c) coating, whilst reducing pressure drop in the air flow. For this reason, it is preferred that at least a selection of the openings of the mesh, grid, or hollow tubes is oriented substantially parallel to the continuous air pathway.

Figures 4(f)&4(g) show grid and mesh support structures (2s) each coated with a layer of catalyst (2c). Any coating technique can be used to apply a coating layer of catalyst (2c) onto the support structure, such as and not limited to dip coating, sputtering, chemical or physical vapour deposition (CVD, PVD), brushing, spraying, and the like.

The air-permeable structure (2p) can form a hollow tubular structure enclosing an elongated source of UV-light (2uv), as illustrated in Figures 3(a) and 4(a). The tubular air-permeable structure can be fixed to a wall of a duct, extending transverse, preferably normal to the continuous air pathway, as shown in Figure 3(a). Alternatively, the air-permeable structure (2p) can be substantially planar, extending over part or the whole cross-section of a duct, with one or more UV-lights irradiating one or both surfaces thereof, as shown in Figure 3(b).

As discussed supra the catalyst (2c) is preferably a semiconductor. The catalyst (2c) can be a metal oxide (MO) selected among one or more of the following semiconductors: TiO₂, WO₂, SnO₂, SrTiO₂, ZnO, WO₃, Fe₂O₃, Cu₂O, CeO₂, ZrO₂, and the like. Other semiconductors, such as ZnS, CdS, MoS₂, or CdSe can be used as catalyst (2c). With a relatively large band gap of 3.2 eV and a UV-spectral region centred around 383 nm, TiO₂ (= titanium dioxide or titania) is preferred for its efficient photoactivity and high stability. The semiconductors and, in particular TiO₂, can be doped to vary their band gaps and spectral regions and enhance their performance. For example, TiO₂ can be doped with one or more of Pt, Au, Ag, Pd, Ru, Rh, In, Li, Na, Mg, Fe, Cr, Ni, Mn, V, Cu, Zn, Co, and the like.

### PEROXIDED AIR (5P)

Ambient air (5a) is drawn through the PCA-inlet (1i) into the PCA-unit (1). The ambient air is at ambient temperature (Ta), ambient pressure (Pa), and ambient relative humidity (RHa). The values of Ta, Pa, and RHa depend on the instant meteorological conditions of the location of the PCA-device (day or night, winter or summer, etc.). The ambient air is then filtered through a filtering section (1f) and pre-conditioned in an air-conditioning section (1ac) to extract a pre-conditioned air (5c) at a pre-conditioned temperature (Tc ≠ Ta) different from the ambient temperature (Ta), at a pre-conditioned pressure (Pc > Pa) greater than the ambient pressure (Pa), and at a pre-conditioned relative humidity (RHc > 0) having a positive value. The pre-conditioned temperature (Tc) can be higher than Ta to heat the aircraft cabin, or lower than Ta to cool it. The pre-conditioned pressure (Pc) must be higher than ambient pressure (Pa) to drive the flow of pre-conditioned air (5c) through the air distribution ducting (3) and into the internal air circulation system of the aircraft

The photo catalytic oxidation unit (2) can be located upstream or downstream of the air conditioning section (1ac). As described supra, the photocatalytic formation of ROS relies on the presence of moisture in the air stream being treated. Depending on the location of the photo catalytic oxidation unit (2), the relative humidity (RHa, RHc) of ambient air (5a) or of the pre-conditioned air (5c) must be non-zero. The photo catalytic oxidation unit (2) is preferably positioned downstream of the air conditioning section (1ac) because in case the ambient air is too dry, the air conditioning section can be equipped with a humidifier to raise the moisture content in the air to optimal levels before it reaches the photo catalytic oxidation unit (2).

The ambient air (5a) or the pre-conditioned air (5c) flows through the photo catalytic oxidation unit (2) where under the combined action of the UV-light and the catalyst undergoes photo catalytic reactions with formation of ROS including dry H₂O₂ (DHP), and other reactive oxygen species such as hydroxyl radicals (˙OH) and superoxides (˙O₂⁻). This is in contrast with the method described in US7354551 which imposes for safety reasons an empty room during and a time after the sanitization treatment with hydrogen peroxide vapour.

DHP concentrations in the air below 1 ppm are considered as safe for humans. The peroxided air (5p) preferably has a dry hydrogen peroxide concentration of not more than 0.06 ppm, preferably not more than 0.04 ppm, and is at least 0.01 ppm, preferably at least 0.02 ppm. Excellent sanitizing results were observed in aircraft cabins at such ranges of DHP contents, with no danger for any material nor any human being present in the cabin during the injection of peroxided air (5p) into the cabin. This means that sanitization of the cabin can be started before the inbound passengers have left the aircraft, proceed during the cleaning operation by the cleaning team, and end after the outbound passengers have boarded the aircraft, thus maximizing the time available for ventilating and sanitizing the aircraft cabin.

### PCA-DEVICE

The PCA-device of the present invention can be obtained from a state-of-the-art PCA-device as illustrated in Figure 5(a), with a simple, yet essential modification, by adding a photo catalytic oxidation unit (2) along the continuous air pathway. As shown in Figures 5(b) to 5(d) discussed supra, the photo catalytic oxidation unit (2) is positioned downstream of the PCA-inlet (1i), either upstream or downstream of the air conditioning section (1 ac). The photo catalytic oxidation unit (2) can be located within the PCA-unit (1), or in the air distribution ducting (3), or at the interface between the PCA-unit (1) and the air distribution ducting (3). The terms *"upstream"* and *"downstream"* used in this document are defined with respect to the flow direction of the air from the PCA inlet (1i) to the coupling head (4).

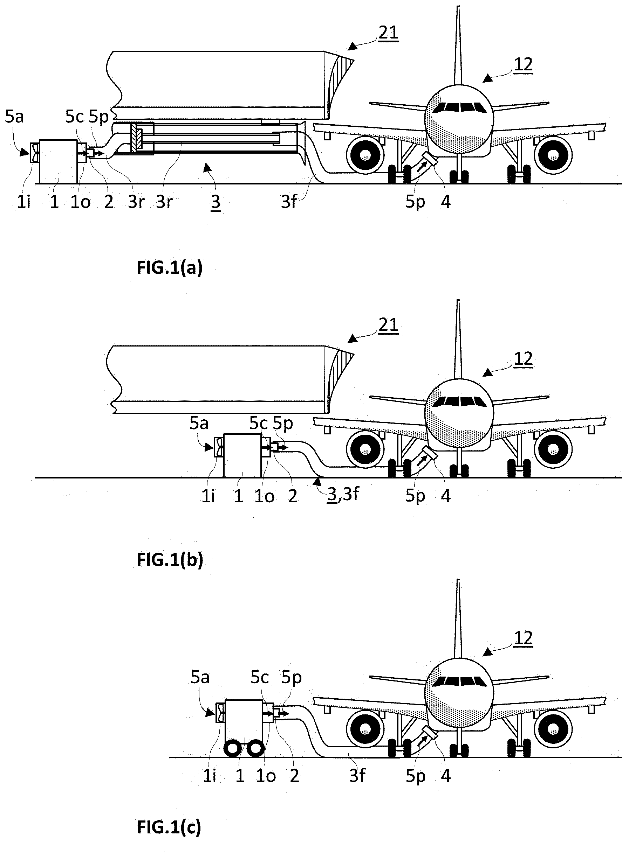

The PCA-device is preferably a combo device, combining pre-conditioned air and electric power supplies as is well known in the art. The electric power is preferably supplied as alternative current at a frequency of preferably 400 Hz. As shown in Figure 1(a) the PCA-unit can be a fixed system coupled to a passenger-boarding bridge (12). Alternatively, the PCA-unit can be a fixed system but independent of a passenger-boarding bridge (12), as shown in Figure 1(b). Finally, the PCA-unit can be a mobile system which can be brought to an aircraft parked at a position remote from a terminal.

### METHOD FOR SANITIZING AN AIR AND SURFACES OF AN AIRCRAFT CABIN

The present invention also concerns a method for ventilating and sanitizing an air and surfaces of an interior of a cabin of a parked aircraft (12) and for conditioning the air in the interior of the cabin. The method comprises the following steps.
- providing a PCA-device as described supra,
- fluidly coupling the coupling head (4) to an internal air circulation duct of the aircraft
- drawing ambient air (5a) into the PCA-device unit (1) through the PCA-inlet (1i),
- conditioning the ambient air inside the PCA-device unit (1) and dispensing a pre-conditioned air stream of pre-conditioned air (5c) out of the PCA-outlet (1o) at the pre-conditioned temperature (Tc), at the pre-conditioned relative humidity (RHc), and at the pre-conditioned pressure (Pc),
- leading the pre-conditioned air stream along the air distribution ducting (3) into the internal air circulation duct of the aircraft,

The method differs from prior art methods for conditioning the air in an aircraft cabin in that it comprises the following steps,
- activating the source of UV-light (2uv) and ensuring that air flowing along the continuous air pathway contacts the catalyst (2c) irradiated by the UV-light within the UV-volume, to form the pre-conditioned air stream of pre-conditioned air (5c) comprising not more than 1 ppm of dry hydrogen peroxide, and
- allowing the peroxided air (5p) to circulate into the cabin for a given time to sanitize and disinfect the air and surfaces it contacts.

Optimal ventilation of the cabin can be ensured by 100%-fresh outside air injection, with no or substantially no recirculation of any air in the cabin, contrary to the air conditioning controlled by the APU. This is also by contrast with air conditioning in buildings which generally recirculate a substantial portion of air.

Preliminary tests at an aircraft manufacturer testing facilities have shown excellent results of sanitization of the air and surfaces of a cabin with a DHP concentration lower than 0.06 ppm with treatment times under 60 min. Even hidden surfaces, such as overhead stowage bins and table trays in the upright position were satisfactorily sanitized. This is made possible because DHP is a gas able to diffuse through very small openings. The low DHP concentrations used ([DHP] < 0.06 ppm) resulted in a safe exposure for humans. In these times of enhanced awareness to elimination of germs and pathogenic microorganisms, such as the coronavirus, the present invention offers a simple, inexpensive, fast, and safe method for reducing considerably the risks of contamination due to contacts with contaminated surfaces in the interior of an aircraft. With the drop in the number of passengers experienced by all airline companies, and the fear of being contaminated, a recognized sanitization, possibly disinfection of the surfaces and air inside an aircraft cabin is a necessary (not sufficient) condition for restoring the confidence of the public towards flying. The PAC-device of the present invention offers a solution to this challenge.

| **Ref** | **Description** |
|---|---|
| 1 | PCA-unit |
| 1ac | Air conditioning section |
| 1f | Filtration section |
| 1i | PCA-inlet |
| 10 | PCA-outlet |
| 2 | Photo catalytic oxidation unit |
| 2c | Catalyst |
| 2p | Air-permeable structure |
| 2s | Support structure of the air-permeable structure |
| 2uv | Source of UV-light |
| 3 | Air duct |
| 3f | Flexible portion of the air duct |
| 3r | Rigid portion of the air duct |
| 4 | Coupling head |
| 5a | Ambient air |
| 5c | Pre-conditioned air |
| 5p | Peroxided air |
| 11 | Aircraft |
| 12 | Passenger-boarding bridge |

## Claims

1. Pre-conditioned air (PCA-) device for conditioning air in an interior of a cabin of a parked aircraft (12) and for sanitizing an air and surfaces in the interior of the cabin, the PCA-device comprising,
• an air pre-conditioning (PCA-) unit (1) comprising,
∘ a PCA-inlet (1i) configured for admitting ambient air (5a) from a surrounding environment, the ambient air being at an ambient temperature (Ta), at an ambient relative humidity (RHa), and an ambient pressure (P),
∘ an air filtration section (1f) located downstream of the PCA-inlet,
∘ an air conditioning section (1ac) located downstream of the PCA-inlet,
∘ a PCA-outlet (1o) located downstream of both air filtration and air conditioning sections, and configured for dispensing a pre-conditioned air stream of pre-conditioned air (5c) at a pre-conditioned temperature (Tc ≠ Ta) different from the ambient temperature (Ta), at a pre-conditioned relative humidity (RHc > 0) having a positive value, and at a pre-conditioned pressure (Pc > Pa) greater than the ambient pressure (Pa),
• an air distribution ducting (3) comprising a flexible portion (3f), the air distribution ducting extending from a proximal end in fluid communication with the PCA-outlet, to a distal end provided with a coupling head (4) configured for being fluidly coupled to an internal air circulation duct of an aircraft,
• wherein the PCA-device defines a continuous air pathway extending from the PCA-inlet (1i) to the coupling head (4),
**characterized in that,** the PCA-device comprises a photo catalytic oxidation unit (2) located downstream of the PCA-inlet (1i), preferably at or downstream of the PCA-outlet (1o) and intersecting the continuous air pathway, the photo catalytic oxidation unit being configured for producing a peroxided air (5p) by formation of dry hydrogen peroxide at a concentration below 1 ppm in the pre-conditioned air (5c) dispensed out of the PCA-outlet, wherein the photo catalytic oxidation unit comprises,
• a source of ultraviolet (UV-) light (2uv) configured for irradiating a UV-volume with UV-light, and
• a catalyst (2c) located within the UV-volume and intersecting the continuous air pathway.

2. PCA-device according to claim 1, wherein the photo catalytic oxidation unit (2) intersects the pre-conditioned air stream of pre-conditioned air (5c) and is preferably located at or directly adjacent to the PCA-outlet (1o).

3. PCA-device according to claim 1 or 2, wherein the catalyst (2c) is coated on a surface of an air-permeable structure (2p) intersecting the continuous air pathway.

4. PCA-device according to the preceding claim, wherein the air-permeable structure (2p) is formed by a mesh or a grid, both comprising openings, or a series of hollow tubes assembled in a honeycomb structure defining openings, wherein at least a selection of the openings of the mesh, grid, or hollow tubes is oriented substantially parallel to the continuous air pathway.

5. PCA-device according to any one of the preceding claims, wherein the catalyst (2c) is a metal oxide, preferably titanium dioxide or tungsten dioxide, or a mixture of metal oxides including titanium dioxide.

6. PCA-device according to any one of the preceding claims, wherein the UV-light has a wavelength comprised between 200 and 400 nm, preferably the UV-light is UV-A light of wavelength comprised between 320 and 400 nm.

7. PCA-device according to any one of the preceding claims, wherein the dry hydrogen peroxide concentration in the peroxided air (5p) is not more than 0.06 ppm, preferably not more than 0.04 ppm, and is at least 0.01 ppm, preferably at least 0.02 ppm.

8. PCA-device according to any one of the preceding claims, wherein the peroxided air (5p) further comprises reactive oxygen species (ROS) including one or more of ˙O₂⁻ and ˙OH.

9. PCA-device according to any one of the preceding claims, wherein the PCA-unit (1) is either,
• a fixed system preferably coupled to a passenger-boarding bridge (12), or
• a mobile system which can be brought to an aircraft parked at a position remote from a terminal.

10. PCA-device according to any one of the preceding claims, wherein the PCA-unit (1) is a combined system configured for also supplying alternative current at a frequency of preferably 400 Hz.

11. Method for sanitizing an air and surfaces of an interior of a cabin of a parked aircraft (12) and for conditioning the air in the interior of the cabin, the method comprising,
• providing a PCA-device according to any one of the preceding claims,
• fluidly coupling the coupling head (4) to an internal air circulation duct of the aircraft
• drawing ambient air (5a) into the PCA-device unit (1) through the PCA-inlet (1i),
• conditioning the ambient air inside the PCA-device unit (1) and dispensing a pre-conditioned air stream of pre-conditioned air (5c) out of the PCA-outlet at the pre-conditioned temperature (Tc), at the pre-conditioned relative humidity (RHc), and at the pre-conditioned pressure (Pc),
• leading the pre-conditioned air stream along the air distribution ducting (3) into the internal air circulation duct of the aircraft,
**characterized in that,** the method comprises the following steps,
• activating the source of UV-light (2uv) and ensuring that air flowing along the continuous air pathway contacts the catalyst (2c) irradiated by the UV-light within the UV-volume, to form the pre-conditioned air stream of pre-conditioned air (5c) comprising not more than 1 ppm of dry hydrogen peroxide, and
• allowing the peroxided air (5p) to circulate into the cabin for a given time to sanitize and disinfect the air and surfaces it contacts.

## Patentansprüche

1. Luftvorkonditionierungsvorrichtung (PCA-Vorrichtung) zum Vorkonditionieren von Luft in einem Innenraum einer Kabine eines geparkten Luftfahrzeugs (12) und zum Hygienisieren von Luft und Oberflächen im Innenraum der Kabine, wobei die PCA-Vorrichtung Folgendes umfasst:
• eine Luftvorkonditionierungseinheit (PCA-Einheit) (1), die Folgendes umfasst:
o einen PCA-Einlass (1i), der zum Einlassen von Umgebungsluft (5a) aus einer umliegenden Umgebung ausgelegt ist, wobei die Umgebungsluft eine Umgebungstemperatur (Ta), eine relative Umgebungsfeuchtigkeit (RHa) und einen Umgebungsdruck (P) aufweist,
o einen Luftfilterabschnitt (1f), der sich stromabwärts des PCA-Einlasses befindet,
o einen Luftkonditionierungsabschnitt (1ac), der sich stromabwärts des PCA-Einlasses befindet,
o einen PCA-Auslass (1o), der sich sowohl stromabwärts des Luftfilterabschnitts als auch des Luftkonditionierungsabschnitts befindet und ausgelegt ist zum Abgeben eines vorkonditionierten Luftstroms vorkonditionierter Luft (5c) bei einer vorkonditionierten Temperatur (Tc ≠ Ta), die sich von der Umgebungstemperatur (Ta) unterscheidet, bei einer vorkonditionierten relativen Feuchtigkeit (RHc > 0) mit einem positiven Wert und bei einem vorkonditionierten Druck (Pc > Pa), der größer als der Umgebungsdruck (Pa) ist,
• eine Luftverteilungsleitung (3), die einen flexiblen Teil (3f) umfasst, wobei sich die Luftverteilungsleitung von einem proximalen Ende in Fluidverbindung mit dem PCA-Auslass zu einem distalen Ende erstreckt, das mit einem Kopplungskopf (4) versehen ist, der zur Fluidkopplung mit einer internen Luftumwälzleitung eines Flugzeugs ausgelegt ist,
• wobei die PCA-Vorrichtung einen kontinuierlichen Luftweg definiert, der sich vom PCA-Einlass (1i) zum Kopplungskopf (4) erstreckt,
**dadurch gekennzeichnet, dass** die PCA-Vorrichtung eine fotokatalytische Oxidationseinheit (2) umfasst, die sich stromabwärts des PCA-Einlasses (1i), vorzugsweise am PCA-Auslass (1o) oder stromabwärts davon, befindet und den kontinuierlichen Luftweg schneidet, wobei die fotokatalytische Oxidationseinheit dazu ausgelegt ist, eine peroxidierte Luft (5p) durch Bildung von trockenem Wasserstoffperoxid mit einer Konzentration unter 1 ppm in der aus dem PCA-Auslass abgegebenen vorkonditionierten Luft (5c) zu erzeugen, wobei die fotokatalytische Oxidationseinheit Folgendes umfasst:
• eine Quelle für ultraviolettes (UV) Licht (2uv), die zum Bestrahlen eines UV-Volumens mit UV-Licht ausgelegt ist, und
• einen Katalysator (2c), der sich innerhalb des UV-Volumens befindet und den kontinuierlichen Luftweg schneidet.

2. PCA-Vorrichtung nach Anspruch 1, wobei die fotokatalytische Oxidationseinheit (2) den vorkonditionierten Luftstrom vorkonditionierter Luft (5c) schneidet und sich vorzugsweise an oder direkt benachbart zu dem PCA-Auslass (1o) befindet.

3. PCA-Vorrichtung nach Anspruch 1 oder 2, wobei der Katalysator (2c) auf eine Oberfläche einer luftdurchlässigen Struktur (2p) aufgetragen ist, die den kontinuierlichen Luftweg schneidet.

4. PCA-Vorrichtung nach dem vorhergehenden Anspruch, wobei die luftdurchlässige Struktur (2p) durch ein Netz oder ein Gitter, die beide Öffnungen umfassen, oder eine Reihe von Hohlrohren gebildet ist, die in einer Wabenstruktur zusammengesetzt ist, die Öffnungen definiert, wobei mindestens eine Auswahl der Öffnungen des Netzes, des Gitters oder der Hohlrohre im Wesentlichen parallel zu dem kontinuierlichen Luftweg ausgerichtet ist.

5. PCA-Vorrichtung nach einem der vorhergehenden Ansprüche, wobei der Katalysator (2c) ein Metalloxid, vorzugsweise Titandioxid oder Wolframdioxid, oder eine Mischung von Metalloxiden ist, die Titandioxid umfasst.

6. PCA-Vorrichtung nach einem der vorhergehenden Ansprüche, wobei das UV-Licht eine Wellenlänge zwischen 200 und 400 nm aufweist, wobei das UV-Licht vorzugsweise UV-A-Licht mit einer Wellenlänge zwischen 320 und 400 nm ist.

7. PCA-Vorrichtung nach einem der vorhergehenden Ansprüche, wobei die Konzentration des trockenen Wasserstoffperoxids in der peroxidierten Luft (5p) nicht mehr als 0,06 ppm, vorzugsweise nicht mehr als 0,04 ppm beträgt und mindestens 0,01 ppm, vorzugsweise mindestens 0,02 ppm beträgt.

8. PCA-Vorrichtung nach einem der vorhergehenden Ansprüche, wobei die peroxidierte Luft (5p) ferner reaktive Sauerstoffspezies (ROS) umfasst, die eines oder mehrere von ˙O₂⁻ und ˙OH umfassen.

9. PCA-Vorrichtung nach einem der vorhergehenden Ansprüche, wobei die PCA-Einheit (1) Folgendes ist:
• ein festes System, das vorzugsweise mit einer Passagiereinstiegsbrücke (12) gekoppelt ist, oder
• ein mobiles System, das von einem Terminal zu einem an einer entfernten Position geparkten Flugzeug gebracht werden kann.

10. PCA-Vorrichtung nach einem der vorhergehenden Ansprüche, wobei die PCA-Einheit (1) ein kombiniertes System ist, das auch zum Liefern von Wechselstrom mit einer Frequenz von vorzugsweise 400 Hz ausgelegt ist.

11. Verfahren zum Hygienisieren von Luft und Oberflächen eines Innenraums einer Kabine eines geparkten Luftfahrzeugs (12) und zum Konditionieren der Luft im Innenraum der Kabine, wobei das Verfahren Folgendes umfasst:
• Bereitstellen einer PCA-Vorrichtung nach einem der vorhergehenden Ansprüche,
• Fluidkoppeln des Kopplungskopfs (4) mit einer internen Luftumwälzleitung des Luftfahrzeugs,
• Ansaugen von Umgebungsluft (5a) in die PCA-Vorrichtungseinheit (1) durch den PCA-Einlass (1i),
• Konditionieren der Umgebungsluft in der PCA-Vorrichtungseinheit (1) und Abgeben eines vorkonditionierten Luftstroms vorkonditionierter Luft (5c) aus dem PCA-Auslass bei der vorkonditionierten Temperatur (Tc), bei der vorkonditionierten relativen Feuchtigkeit (RHc) und bei dem vorkonditionierten Druck (Pc),
• Führen des vorkonditionierten Luftstroms entlang der Luftverteilungsleitung (3) in die interne Luftumwälzleitung des Luftfahrzeugs,
**dadurch gekennzeichnet, dass** das Verfahren die folgenden Schritte umfasst:
• Aktivieren der Quelle für UV-Licht (2uv) und Sicherstellen, dass Luft, die entlang des kontinuierlichen Luftwegs strömt, mit dem Katalysator (2c) in Kontakt kommt, der von dem UV-Licht innerhalb des UV-Volumens bestrahlt wird, um den vorkonditionierten Luftstrom vorkonditionierter Luft (5c) zu bilden, der nicht mehr als 1 ppm trockenes Wasserstoffperoxid umfasst, und
• Zirkulierenlassen der peroxidierten Luft (5p) für eine bestimmte Zeit in die Kabine, um die Luft und die Oberflächen, mit denen sie in Kontakt kommt, zu hygienisieren und zu desinfizieren.

## Revendications

1. Dispositif d'air préconditionné (PCA) destiné à conditionner l'air à l'intérieur d'une cabine d'un aéronef parqué (12) et à assainir l'air et les surfaces à l'intérieur de la cabine, le dispositif PCA comprenant
• une unité de préconditionnement d'air (PCA) (1) comprenant
∘ une entrée PCA (1i) conçue pour laisser entrer de l'air ambiant (5a) depuis un milieu environnant, l'air ambiant étant à une température ambiante (Ta), à une humidité relative ambiante (RHa), et à une pression ambiante (P),
o une section de filtration d'air (1f) située en aval de l'entrée PCA,
o une section de conditionnement d'air (1ac) située en aval de l'entrée PCA,
o une sortie PCA (1o) située en aval des sections à la fois de filtration d'air et de conditionnement d'air, et conçue pour délivrer un courant d'air préconditionné d'air préconditionné (5c) à une température préconditionnée (Tc ≠ Ta) différente de la température ambiante (Ta), à une humidité relative préconditionnée (RHc > 0) ayant une valeur positive, et à une pression préconditionnée (Pc > Pa) supérieure à la pression ambiante (Pa),
• une gaine de distribution d'air (3) comprenant une partie flexible (3f), la gaine de distribution d'air s'étendant depuis une extrémité proximale en communication fluidique avec la sortie PCA jusqu'à une extrémité distale pourvue d'une tête de couplage (4) conçue pour être couplée fluidiquement à une gaine de circulation d'air interne d'un aéronef,
• le dispositif PCA définissant un passage d'air continu s'étendant de l'entrée PCA (1i) jusqu'à la tête de couplage (4),
**caractérisé en ce que** le dispositif PCA comprend une unité d'oxydation photocatalytique (2) située en aval de l'entrée PCA (1i), de préférence au niveau ou en aval de la sortie PCA (1o) et croisant le passage d'air continu, l'unité d'oxydation photocatalytique étant conçue pour produire un air peroxydé (5p) par formation de peroxyde d'hydrogène sec à une concentration inférieure à 1 ppm dans l'air préconditionné (5c) délivré par la sortie PCA, dans lequel l'unité d'oxydation photocatalytique comprend
• une source de rayonnement ultraviolet (UV) (2uv) conçue pour irradier un volume UV avec un rayonnement UV, et
• un catalyseur (2c) situé à l'intérieur du volume UV et croisant le passage d'air continu.

2. Dispositif PCA selon la revendication 1, dans lequel l'unité d'oxydation photocatalytique (2) croise le courant d'air préconditionné d'air préconditionné (5c) et est de préférence située au niveau ou au voisinage direct de la sortie PCA (1o).

3. Dispositif PCA selon la revendication 1 ou 2, dans lequel le catalyseur (2c) est déposé sur une surface d'une structure perméable à l'air (2p) croisant le passage d'air continu.

4. Dispositif PCA selon la revendication précédente, dans lequel la structure perméable à l'air (2p) est formée par un treillis ou une grille, tous deux comprenant des ouvertures, ou une série de tubes creux assemblés en une structure en nid d'abeilles définissant des ouvertures, dans lequel au moins une sélection des ouvertures du treillis, de la grille ou des tubes creux est orientée de façon sensiblement parallèle au passage d'air continu.

5. Dispositif PCA selon l'une quelconque des revendications précédentes, dans lequel le catalyseur (2c) est un oxyde métallique, de préférence du dioxyde de titane ou du dioxyde de tungstène, ou un mélange d'oxydes métalliques comportant du dioxyde de titane.

6. Dispositif PCA selon l'une quelconque des revendications précédentes, dans lequel le rayonnement UV a une longueur d'onde comprise entre 200 et 400 nm, de préférence le rayonnement UV est un rayonnement UV-A de longueur d'onde comprise entre 320 et 400 nm.

7. Dispositif PCA selon l'une quelconque des revendications précédentes, dans lequel la concentration de peroxyde d'hydrogène sec dans l'air peroxydé (5p) ne dépasse pas 0,06 ppm, de préférence ne dépasse pas 0,04 ppm, et est d'au moins 0,01 ppm, de préférence d'au moins 0,02 ppm.

8. Dispositif PCA selon l'une quelconque des revendications précédentes, dans lequel l'air peroxydé (5p) comprend en outre des espèces réactives de l'oxygène (ROS) dont une ou plusieurs parmi ˙O₂⁻ et ˙OH.

9. Dispositif PCA selon l'une quelconque des revendications précédentes, dans lequel l'unité PCA (1) est
• un système fixe de préférence couplé à une passerelle d'embarquement (12), ou
• un système mobile qui peut être amené jusqu'à un aéronef parqué à une position distante d'un terminal.

10. Dispositif PCA selon l'une quelconque des revendications précédentes, dans lequel l'unité PCA (1) est un système combiné conçu pour fournir également un courant alternatif à une fréquence de préférence de 400 Hz.

11. Procédé destiné à assainir l'air et les surfaces de l'intérieur d'une cabine d'un aéronef parqué (12) et à conditionner l'air à l'intérieur de la cabine, le procédé comprenant
• la fourniture d'un dispositif PCA selon l'une quelconque des revendications précédentes,
• le couplage fluidique de la tête de couplage (4) à une gaine de circulation d'air interne de l'aéronef,
• l'aspiration d'air ambiant (5a) à l'intérieur de l'unité de dispositif PCA (1) par l'entrée PCA (1i),
• le conditionnement de l'air ambiant à l'intérieur de l'unité de dispositif PCA (1) et la délivrance d'un courant d'air préconditionné d'air préconditionné (5c) par la sortie PCA à la température préconditionnée (Tc), à l'humidité relative préconditionnée (RHc), et à la pression préconditionnée (Pc),
• l'acheminement du courant d'air préconditionné le long de la gaine de distribution d'air (3) jusqu'à l'intérieur de la gaine de circulation d'air interne de l'aéronef,
**caractérisé en ce que** le procédé comprend les étapes suivantes :
• activer la source de rayonnement UV (2uv) en s'assurant que l'air circulant le long du passage continu d'air entre en contact avec le catalyseur (2c) irradié par le rayonnement UV à l'intérieur du volume UV, pour former le courant d'air préconditionné d'air préconditionné (5c) ne comprenant pas plus de 1 ppm de peroxyde d'hydrogène sec, et
• laisser l'air peroxydé (5p) circuler à l'intérieur de la cabine pendant un temps donné pour assainir et désinfecter l'air et les surfaces avec lesquels il entre en contact.
